(19) Europäisches Patentamt
European Patent Office
Office européen des brevets

(11) Publication number: 0 247 614 B1

# (12) EUROPEAN PATENT SPECIFICATION

(45) Date of publication of patent specification: 09.01.91 Bulletin 91/02

(51) Int. Cl.[5]: C07C 29/124, C07C 31/42, C07C 31/38

(21) Application number: 87107758.2

(22) Date of filing: 27.05.87

## (54) Process for the synthesis of mono-or di-hydroxyfluoroalkanes.

(30) Priority: 30.05.86 IT 2065586

(43) Date of publication of application: 02.12.87 Bulletin 87/49

(45) Publication of the grant of the patent: 09.01.91 Bulletin 91/02

(84) Designated Contracting States: BE DE ES FR GB IT NL SE

(56) References cited:
EP-A- 0 008 096
EP-A- 0 089 859
DE-A- 2 318 941
DE-A- 3 016 571
DE-A- 3 035 641
FR-A- 2 486 521
US-A- 3 171 861
US-A- 3 283 012
US-A- 4 590 310

(73) Proprietor: AUSIMONT S.r.l.
Foro Buonaparte 31
I-20121 Milano (IT)

(72) Inventor: Bargigia, Gianangelo
47, via Federico Chopin
I-20141 Milan (IT)
Inventor: Tonelli, Claudio
25, via Gino Valagussa
I-20049 Concorezzo Milan (IT)
Inventor: Tortelli, Vito
46, viale Corsica
I-20137 Milan (IT)

(74) Representative: Barz, Peter, Dr. et al
Patentanwälte Dipl.-Ing. G. Dannenberg Dr. P. Weinhold, Dr. D. Gudel Dipl.-Ing. S. Schubert, Dr. P. Barz Siegfriedstrasse 8
D-8000 München 40 (DE)

EP 0 247 614 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

The present invention relates to the preparation of mono- and di-hydroxyfluoroalkanes starting from the corresponding iodo-fluoroalkanes.

In known processes for the synthesis of fluoroalkanols of the above-mentioned type starting from the corresponding iodides, for example oleum is used as a hydrolizing agent (US-A- 3 283 012). This has the serious drawback that removal of a considerable excess of $H_2SO_4$ is required. Furthermore, the recovery of iodine is complex and by-products such as ethers or olefins may easily be formed.

Another process (DE-A- 3 016 517) is based on a reaction in a two-phase liquid system, comprising a phase transfer catalyst and/or perfluorocarboxylic surfactants. The process comprises the step of reacting fluoroiodoalkanes with a stoichiometric amount or with an excess of salts of Cu, Ag or Hg.

A drawback in this method is the possible forming of emulsions which are difficult to handle, the use of large amounts of the above-said salts which must be recovered, as well as of expensive ingredients such as the phase transfer catalyst.

DE-A- 3 035 641 discloses a process for the preparation of alkanols by means of a reaction between iodides and peroxyacids, the alkanols yield being equal to 86.5%.

The object of the present invention is a new process for preparing hydroxyfluoroalkanes of the general formula :

$$X - R_f - Y \qquad (I).$$

wherein X and Y, equal or different from each other, are -F or $-CH_2CH(A)OH$ (A is H or alkyl of 1 to 3 carbon atoms), X being different from F when Y is F and vice-versa ; $R_f$ is a straight or branched perfluoroalkylene chain containing 2 to 18 carbon atoms, preferably 4 to 12 carbon atoms.

The process comprises reacting in a homogeneous phase compounds of formula :

$$W - R_f - T \qquad (II)$$

where $R_f$ is as defined above and W and T, equal or different from each other, are -F, $-CH_2CH(A)I$ (A is H or alkyl of 1 to 3 carbon atoms), W being different from F when T is F and vice-versa, with water in an organic solvent, completely miscible with water or in which water is soluble at 1.7% at least by weight at 20°C, in the presence of a catalyst comprising one or more transition metal ions. Preferred metal ions are those having an atomic number from 24 to 29, from 44 to 47, from 74 to 79 and those having an atomic number of 42 and 58. Particularly preferred are Cu, Fe, Co, Ni, Ag.

Useful transition metal compounds are hydrated salts and oxides, both those which are fully soluble and those which are little soluble in the reaction medium. Preferred compounds are the ones whose anion has low basic and nucleophile properties, for example, sulphates, nitrates, phosphates and fluoborates.

Preferred organic solvents are $CH_3CN$, DMF, DMSO, $CH_3COOH$, $CH_3NO_2$, in particular $CH_3CN$, DMF, DMSO.

The process temperature ranges from 120 to 280°C, preferably from 140 to 200°C.

The time required depends on the specific reagents and on the temperature. Generally it is of the order of a few hours. Longer times, even much longer times, do not detrimentally affect the reaction selectivity.

The reaction takes place under an autogenous pressure.

The metal concentration expressed as gram atoms of metal/gram atoms of iodine ratio is lower than 0.1, preferably lower than 0.01 and at least 0.0005.

The amount of $H_2O$ expressed as mols of $H_2O$/gram atoms of iodine ratio ranges from 1 to 100, preferably from 2 to 30.

The $H_2O$/organic solvent volume ratio preferably ranges from 0.1 to 1.2.

Examples of suitable iodofluoroalkanes are the ones obtained by addition of ethylene or alpha-olefins to the iodoperfluoroalkanes described in IT-A- 19 652 A/85 and 20 235 A/85, US-A- 3 514 487 and DE-A- 2 054 922.

The yields obtained through the reaction carried out according to the process of the invention are very high, generally at least 95%. Also the selectivity is very high. In particular, the dehydrohalogenation products having unsaturated end groups are either absent or present only as traces.

The process of the invention utilizes metal ions which do not cause pollution problems and are inexpensive. Said metal ions are used in small amounts, that being a further improvement with respect to the prior

art, according to which, for similar reactions, metal ion salts were utilized in stoichiometric amounts.

Another advantage of the process of the present invention is that iodine, which is contained in the starting compounds, after the reaction is present as hydroiodic acid, which is easy to recover, what results in an economic advantage.

When the starting compound has an end group of formula

$$\begin{array}{l} -CF-CH_2-CH_2-I, \\ \;\;| \\ \;CF_3 \end{array}$$

it is possible to keep unaltered this group and to substitute only the other iodine atom by a hydroxy group, thus obtaining a product, which is utilizable for subsequent asymmetric reactions.

A particular feature of the present invention which represents still a further advantage with respect to the art resides in the fact that it is possible to synthesize the compounds of formula (II) directly in the reaction medium of the process of the invention, by addition of ethylene or alpha-olefins to iodoperfluoroalkanes.

The hydroxyfluoroalkanes prepared according to the process of the invention are useful as intermediates for preparing products utilized in the treatment of textiles in order to impart oil- and water-repellency to them.

In particular, said hydroxyfluoroalkanes are converted to esters with unsaturated carboxylic acids, and then are polymerized.

The following examples are given to illustrate the present invention, but are not be considered as a limitation thereof.

Example 1 - Two-step synthesis

A) Precursor preparation.

Into a 5-liter pressure reactor AISI 316 were charged 1.15 kg of $C_4F_8I_2$ (2.53 moles) and 25.2 g of CuI (0.10 moles) in a 2 l of acetonitrile. The reactor was closed and purged with $N_2$. The pressure was brought to 64 atm by means of ethylene. The temperature was gradually raised to 160°C and the whole was allowed to stand for 8 hours. After cooling to room temperature, excess ethylene was discharged, the reactor was then opened and water was added until a white precipitate was obtained. This was filtered and vacuum-dried at 70°C. The crude precipitate was purified by crystallization from hexane, yielding 1.26 kg (98%) of product. On the basis of the analytical data (G.C., N.M.R $^{19}F$, N.M.R $^{1}H$) the product proved to be :

$$ICH_2CH_2(CF_2)_4CH_2CH_2I.$$

B) Hydrolysis reaction.

Into an Inconel 5-liter autoclave 1.00 kg (1.96 moles) of the product obtained in step A) was charged with 16.35 g (0.066 moles) of $CuSO_4 \cdot 5H_2O$, 1.8 l of acetonitrile and 1 liter of $H_2O$. After heating to 160°C under intense stirring, this temperature was maintained for about 12 hours. After cooling to room temperature, the reactor content was treated with excess water until complete separation of a heavy phase. The mother liquors were repeatedly extracted with ethyl ether which, in turn, was combined with the preceding phase. The solvents were removed under reduced pressure. The residue was treated with 200 ml of 10% NaOH at 70°C for 2 hours. The organic phase was separated and washed with water. The mother liquors, in turn, were extracted with ether. The ether phase was combined with the previously separated organic phase.

Ether was separated by fractioned distillation. The residue was distilled under reduced pressure giving 540 g (96%) of a white crystalline solid which, according to its analytical data (NMR $^{19}F$, NMR $^{1}H$) proved to be:

$$HOCH_2CH_2CF_2CF_2\overset{d}{C}F_2\overset{c}{C}F_2\overset{b}{C}H_2\overset{a}{C}H_2OH,$$

the chemical shift values (relative to TMS and $CCl_3F$) being as follows :

a = 3.9 ppm (triplet)

b = 2.3 - 2,6 ppm (multiplet)

c = -113.5 ppm

d = -123.5 ppm

On G.C. analysis, the product revealed one peak only.

Example 2 - One-step synthesis

Into the same reactor of example 1, step B) and following the same working conditions, 870 g (1.57 moles) of $C_6F_{12}I_2$ were charged, together with 15.6 g (0.063 moles) of $CuSO_4{\cdot}5H_2O$, 1.8 l of acetonitrile, and 1 l of water. Ethylene was then introduced until a pressure of 52 atm was reached.

The whole was gradually brought to 160°C, and this temperature was maintained for 12 hours under intense stirring. It was cooled down to room temperature, and following the procedure of example 1, step B), 585 g (95.6%) of a crystalline solid were obtained, which according to its analytical data (NMR $^1H$, NMR $^{19}F$), proved to be :

$$HOCH_2CH_2CF_2CF_2CF_2\overset{e}{C}F_2\overset{d}{C}F_2\overset{c}{C}F_2\overset{b}{C}H_2\overset{a}{C}H_2OH,$$

the chemical shift values (relative to TMS and $CCl_3F$) being :

a = 3.9 ppm (triplet)

b = 2.3-2.6 ppm (multiplet)

c = -113.5 ppm

d = -123.5 ppm

e = -121.5 ppm.

G.C. analysis revealed the presence of a single peak.

Example 3

Into the reactor of example 1, step B) and according to the same working conditions, 900 g (1.47 moles) of a mixture of $C_6F_{12}I_2$ (40%) and $C_8F_{16}I_2$ (60%) were charged, together with 14.6 g (0.059 moles) of $CuSO_4{\cdot}5H_2O$, 1.8 l of acetonitrile and 1 l of $H_2O$. Ethylene was then introduced until a pressure of 50 atm was attained.

The reactor was gradually brought to 165°C and maintained at this temperature for about 15 hours. After cooling to room temperature, the procedure of Example 1, step B) was followed. After crystallization from hexane, 640 g (98%) of a white solid product was obtained. Gaschromatographic analyses showed two peaks (the areas of which were in the ratio of 2 :3 to each other), the first of which corresponded to the product of Example 2. From the spectroscopic analysis (NMR $^{19}F$, NMR $^1H$) the following composition of the final product was obtained :

40% $HOCH_2CH_2CF_2CF_2CF_2CF_2CF_2CF_2CH_2CH_2OH$,

f e d c b a

60% $HOCH_2CH_2CF_2CF_2CF_2CF_2CF_2CF_2CF_2CF_2CH_2CH_2OH$,

the chemical shift values (relative to TMS and $CCl_3F$) being :

| | | | |
|---|---|---|---|
| a | = | 3.9 | ppm (triplet) |
| b | = | 2.3-2.6 | ppm (multiplet) |
| c | = | -113.5 | ppm |
| d | = | -123.5 | ppm |
| e,f | = | -121.5 | ppm. |

Example 4

Into a Monel pressure reactor of 250 ml volume, 50 g (0.083 moles) of

$$I(CF_2)_5\underset{\displaystyle CF_3}{\underset{|}{C}}FI$$

were introduced together with 0.825 g (0.0033 moles) of $CuSO_4 \cdot 5H_2O$ in a 110 ml of acetonitrile and 30 ml of water.

The reactor was closed and air present therein was let off. Ethylene was introduced up to a pressure of 60 atm. The reactor was brought to 180°C and allowed to stand for about 15 hours under intense stirring. After cooling down to room temperature and, operating as described in Example 1, step B), a colourless liquid product was obtained which, on gaschromatographic and spectrographic analyses (NMR $^{19}F$ and NMR $^{1}H$), proved to be composed of :

m l i h g f e d $CF_3$

14% $HOCH_2CH_2CF_2CF_2CF_2CF_2CF_2\underset{|}{C}FCH_2CH_2I$ (with $CF_3$ on the CF carbon)

c b a

a = 3.4 ppm (triplet)

b = 2.8-3.1 ppm (multiplet)

c = -182.5 ppm

d = -75 ppm

e = -117 ppm

f,g = -121.5 ppm

h = -123.5 ppm

i = -113.5 ppm

l = 2.3-2.6 ppm (multiplet)

m = 3.9 ppm (triplet)

$$86\%\quad HOCH_2CH_2CF_2CF_2CF_2CF_2CF_2CF(\overset{d}{C}F_3)CH_2CH_2OH,$$

m l i h g f e c b a

a = 3.75 ppm (triplet)

b = 2.1-2.4 ppm (multiplet)

c = -186.5 ppm

d = -76 ppm

e = -117 ppm

f,g = -121.5 ppm

h = -123.5 ppm

i = -113.5 ppm

l = 2.3-2.6 ppm (multiplet)

m = 3.9 ppm (triplet)

## Example 5

Into the reactor of example 4 and according to the same operative modalities 23 g (0.05 moles) of $C_4F_8I_2$ were introduced together with 0.64 g (0.0023 moles) of $FeSO_4 \cdot 7H_2O$, 60 ml of $CH_3CN$ and 40 ml of water. Ethylene was introduced until a pressure of 60 atm was attained. It was heated to 160°C for 12 hours under stirring. The product was recovered as in Example 4 yielding 13.9 g (96%) of a white solid product which, according to GC and NMR analyses, proved to be :

$$HOCH_2CH_2(CF_2)_4\ CH_2CH_2OH.$$

Example 6

Into an Inconel 250 ml pressure reactor, 31 g (0.06 moles) of $ICH_2CH_2(CF_2)_4CH_2CH_2I$ were charged together with 0.920 g (0.0037 moles) of $CuSO_4.5H_2O$, 60 ml of dimethylsulphoxide and 40 ml of water. The reactor was closed and purged with $N_2$. Then it was heated to 165°C for 14 hours under stirring. By operating according to the procedure described in Example 1, step B), with the exception of the treatment with soda, 16.7 g (96%) of a white solid product was obtained which, according to its analytical data (GC and NMR), proved to be :

$$HOCH_2CH_2(CF_2)_4\ CH_2CH_2OH.$$

The head fraction contained also traces of olefin :

$$HOCH_2CH_2(CF_2)_4\ CH_2{=}CH_2.$$

Example 7

By repeating example 6, with the exception that instead of dimethylsulphoxide, an equal amount of N,N-dimethylformamide wa used, 16.9 g (97%) of the following product were obtained :

$$HOCH_2CH_2(CF_2)_4\ CH_2CH_2OH.$$

Example 8

By repeating Example 6, with the exception that instead of dimethylsulphoxide, an equal amount of acetic acid was used, 16.3 g (94%) of the following product were obtained :

$$HOCH_2CH_2(CF_2)_4\ CH_2CH_2OH.$$

Example 9

By repeating Example 6, with the exception that instead of dimethylsulphoxide, an equal amount of nitromethane was used, 16.6 g (95%) of the following product were obtained :

$$HOCH_2CH_2(CF_2)_4\ CH_2CH_2OH.$$

Example 10

4.8 g (0.0078 moles) of $I\text{-}CH_2\text{-}CH_2\text{-}(CF_2)_6\text{-}CH_2\text{-}CH_2\text{-}I$ were mixed in a sealed glass reactor with 8 ml of acetonitrile, 8 ml of $H_2O$ and 80 mg of silver acetate. The reactor was then purged with $N_2$, and the temperature was brought to 165°C and maintained at that level for 16 hours. After cooling down and reaching room temperature, the reaction was carried out as in Example 1, step B).
According to IR and NMR analyses, the product obtained consisted of :

$$HOCH_2CH_2(CF_2)_6CH_2CH_2OH \quad 90\% \text{ mol-}\%$$

$$HOCH_2CH_2(CF_2)_6CH_2CH_2I \quad 10\% \text{ mol-}\%$$

## Claims

1. A process for preparing hydroxyfluoroalkanes of the general formula :

$$X - R_f - Y \qquad (I)$$

wherein X and Y, equal or different from each other, are -F or $-CH_2CH(A)OH$ (A is H or alkyl of 1 to 3 carbon atoms), X being different from F when Y is F, and vice-versa, $R_f$ is a straight or branched perfluoroalkylene chain containing 2 to 18 carbon atoms, which comprises, reacting in a homogeneous phase, compounds of formula :

$$W - R_f - T \qquad (II)$$

where $R_f$ is as defined above and W and T, equal or different from each other, are -F, $-CH_2CH(A)I$ (A is H or alkyl of 1 to 3 carbon atoms), W being different from F when T is F, and vice-versa, with water in an organic solvent, which is fully miscible with water or in which water is soluble at 1.7% at least by weight at 20°C, in the presence of a catalyst comprising one or more transition metal ions selected from those having an atomic number ranging from 24 to 29, from 44 to 47, from 74 to 79, or the atomic number 42 or 58.

2. A process according to claim 1, wherein the metal is selected from Cu, Fe, Co, Ni and Ag.

3. A process according to claim 1 or 2, wherein the reaction temperature ranges from 120 to 280°C.

4. A process according to any one of claims 1 to 3, wherein the organic solvent is selected from $CH_3CN$, DMF, DMSO, $CH_3COOH$ and $CH_3NO_2$.

5. A process according to any one of claims 1 to 4, wherein the compounds of formula (II) are directly synthesized in the reaction medium by addition of ethylene or of alphaolefins to the corresponding iodoperfluoroalkanes.

## Ansprüche

1. Verfahren zur Herstellung von Hydroxyfluoralkanen der allgemeinen Formel :

$$X - R_f - Y \qquad (I)$$

in der X und Y gleich oder verschieden voneinander -F oder $-CH_2CH(A)OH$ (A ist H oder Alkyl mit 1 bis 3 Kohlenstoffatomen) bedeuten, wobei X verschieden von F ist, wenn Y F ist, und umgekehrt, $R_f$ eine geradkettige oder verzweigte Perfluoralkylenkette mit 2 bis 18 Kohlenstoffatomen ist, welches umfaßt die Umsetzung in homogener Phase von Verbindungen der Formel :

$$W - R_f - T \qquad (II)$$

in der $R_f$ wie oben definiert ist und W und T gleich oder verschieden voneinander -F, $-CH_2CH(A)I$ (A ist H oder Alkyl mit 1 bis 3 Kohlenstoffatomen) bedeuten, W verschieden von F ist, wenn T F ist, und umgekehrt,

mit Wasser in einem organischen Lösungsmittel, das mit Wasser vollständig mischbar ist oder in dem Wasser zu mindestens 1,7 Gewichtsprozent bei 20°C löslich ist, in Gegenwart eines Katalysators, der ein oder mehrere Übergangsmetallionen umfaßt, ausgewählt aus jenen mit einer Atomzahl von 24 bis 29, von 44 bis 47, von 74 bis 79 oder der Atomzahl 42 oder 58.

2. Verfahren nach Anspruch 1, worin das Metall ausgewählt ist unter Cu, Fe, Co, Ni oder Ag.

3. Verfahren nach Anspruch 1 oder 2, worin die Reaktionstemperatur im Bereich von 120 bis 280°C liegt.

4. Verfahren nach irgendeinem der Ansprüche 1 bis 3, worin das organische Lösungsmittel ausgewählt ist unter $CH_3CN$, DMF, DMSO, $CH_3COOH$ und $CH_3NO_2$.

5. Verfahren nach irgendeinem der Ansprüche 1 bis 4, worin die Verbindungen der Formel (II) direkt in dem Reaktionsmedium synthetisiert werden durch Zugabe von Ethylen oder von α-Olefinen zu den entsprechenden Iodperfluoralkanen.

## Revendications

1. Un procédé pour préparer des hydroxyfluoroalcanes de formule générale :

$$X - R_f - Y \qquad (I)$$

dans laquelle X et Y sont identiques ou différents et sont chacun un atome de fluor ou un radical -$CH_2CH(A)OH$ (A est un atome d'hydrogène ou un groupe alkyle ayant 1 à 3 atomes de carbone), X étant différent d'un atome de fluor lorsque Y est un atome de fluor et vice-versa ; Rf est une chaîne perfluoroalkylène droite ou ramifiée contenant 2 à 18 atomes de carbone, caractérisé en ce qu'il comprend la réaction en phase homogène, de composés de formule :

$$W - R_f - T \qquad (II)$$

dans laquelle $R_f$ est tel que défini plus haut, et W et T sont identiques ou différents et représentent chacun un atome de fluor ou un radical -$CH_2CH(A)$ I (où A est un atome d'hydrogène ou un groupe alkyle ayant 1 à 3 atomes de carbone), W étant différent d'un atome de fluor lorsque T est un atome de fluor et vice-versa, avec de l'eau dans un solvant organique, qui est complètement miscible à l'eau ou dans lequel l'eau est soluble à 1,7% au moins en poids à 20°C, en présence d'un catalyseur comprenant un ou plusieurs ions de métaux de transition choisis parmi ceux qui ont un numéro atomique allant de 24 à 29, de 44 à 47, de 74 à 79 ou le numéro atomique 42 et 58.

2. Un procédé suivant la revendication 1, caractérisé en ce que le métal est choisi parmi Cu, Fe, Co, Ni et Ag.

3. Un procédé suivant la revendication 1 ou la revendication 2, caractérisé en ce que la température de réaction est de l'ordre de 120 à 280°C.

4. Un procédé suivant l'une quelconque des revendications 1 à 3, caractérisé en ce que le solvant organique est choisi parmi $CH_3CN$, DMF, DMSO, $CH_3COOH$ et $CH_3NO_2$.

5. Un procédé suivant l'une quelconque des revendications 1 à 3, caractérisé en ce que les composés de formule (II) sont synthétisés directement dans le milieu réactionnel par addition d'éthylène ou d'alpha-oléfines aux iodoperfluoroalcanes correspondants.